# EUROPEAN PATENT APPLICATION

(11) **EP 4 464 658 A2**
(43) Date of publication of application: **20.11.2024**
(21) Application number: 23740123.7
(22) Date of filing: 10.03.2023
(51) Int. Cl.: B82Y 5/00, B82Y 40/00, A61K 39/39, A61K 39/385, A61P 37/04

(54) **ALUMINUM NANOCRYSTAL DELIVERY SYSTEM, AND SELF-ASSEMBLED PARTICLE ADJUVANT VACCINE BASED ON BINDING OF ALUMINUM NANOCRYSTAL DELIVERY SYSTEM AND VACCINE ANTIGEN MOLECULE**

(30) Priority: 12.01.2022 CN 202210032960
(71) Applicant: Guangzhou Realbenefitspot Pharmaceutical Co., Ltd., Guangzhou, Guangdong 510663 (CN)
(72) Inventor: WANG, Yaling, Guangzhou, Guangdong 510535 (CN); CHEN, Chunying, Guangzhou, Guangdong 510535 (CN); ZHAO, Yuliang, Guangzhou, Guangdong 510535 (CN)
(74) Representative: J A Kemp LLP
(86) International application number: PCT/CN2023/080897
(87) International publication number: WO 2023/134783

(57) **Abstract**

The present invention relates to the technical field of biomedicine technology and vaccines, and particularly relates to an aluminum nanocrystal delivery system with a surface covered with an Fc affinity protein and a preparation method for a self-assembled particle adjuvant vaccine. An aluminum nanocrystal is used as a carrier, the surface of the aluminum nanocrystal is covered with an Fc affinity protein molecular layer and an antigen molecule, and the antigen of a recombinant Fc Tag specifically binds to an Fc affinity protein, so that antigen self-assembly is realized, a virus-like particle vaccine is formed, and the antigen density is improved. The present vaccine can generate a high-titer specific antibody by inducing a body fluid and cell immunity.

## Description

### TECHNICAL FIELD

The present invention relates to the technical field of biomedicine technology and vaccines, and particularly relates to an aluminum nanocrystal vaccine delivery system and a preparation method thereof, and a self-assembled particle adjuvant vaccine based on specific binding of an Fc affinity protein covering a surface of an aluminum nanocrystal in the aluminum nanocrystal vaccine delivery system and a vaccine antigen molecule.

### BACKGROUND

Adjuvants are crucial to the efficacy of subunit vaccines. Since Glenny and his colleagues first discovered in 1926 that combining aluminum salts (KAl(SO₄)₂·12H₂O) with diphtheria toxoid could increase vaccine potency, aluminum salt adjuvants had become the only vaccine adjuvants for human and were widely used in vaccine preparations. Existing aluminum salt adjuvants include potassium aluminum sulfate, aluminum phosphate, and aluminum hydroxide. The aluminum hydroxide adjuvant is the most widely used. It can greatly improve the immunogenicity of vaccines.

The specific surface area and uniformity of the aluminum hydroxide adjuvant determine the adsorption efficiency of the aluminum adjuvant to antigens. The smaller the aluminum hydroxide adjuvant particles, the larger the specific surface area, the more antigens can be adsorbed, and the more conducive to improving the immunogenicity of the vaccine. The existing aluminum hydroxide adjuvant generally activates humoral immunity but rarely T cell-mediated immunity, which is related to the presentation mode of adsorbed antigen of the aluminum hydroxide adjuvant. In addition to adjuvants, the antigen density, intracellular presentation level, and spatiotemporal characteristics also play an extremely important role in the production of antibodies (Nature Biomedical Engineering 2020, 4(6), 636-648.Advanced Drug Delivery Reviews 2020, 158, 91-115.). After the micron-sized aluminum hydroxide adjuvant adsorbs the antigens, the aluminum adjuvant generally binds to the cell membranes of antigen-presenting cells such as dendritic cells (DC cells) to induce the formation of lipid rafts, causing the activation of downstream pathways, which cannot interfere with the fate of the presented antigens in the cell. The antigens are presented by the major histocompatibility complex II (MHC-II) after being processed by a lysosomal pathway, rather than by the cross-presentation of the major histocompatibility complex I (MHC-I). Nanocrystallization of the aluminum hydroxide adjuvant may change the uptake by antigen-presenting cells, thereby affecting the presentation mode of its adsorbed antigens. Although administration by adsorbing the antigens by the aluminum adjuvant is the most common vaccine combination mode, in the presence of serum or interstitial fluid, the surface adsorbed antigens will be rapidly desorbed from the aluminum adjuvant surface due to competitive adsorption, affecting the immune response ability of the vaccine (Nature Medicine.2020, 26(3), 430-440.). Moreover, some antigens are too strongly adsorbed by aluminum adjuvants, resulting in changes in antigen conformation, thereby slowing down and affecting the production of antigen-specific antibodies. Although there are some limitations in the application process of aluminum adjuvants, due to the strict requirements for vaccine safety, the successful development of novel adjuvants requires a long process and huge financial investment. Therefore, it will be of great practical value to develop a universal delivery platform for subunit vaccines based on aluminum adjuvant particles to reduce the impact of nonspecific adsorption of antigen adjuvants on antigens and increase the dose of antigens taken up by antigen-presenting cells. The inventors aim to disclose a structurally stable aluminum nanocrystal delivery system and a preparation method for a self-assembled particle adjuvant vaccine based on specific binding of an Fc affinity protein covering a surface of an aluminum nanocrystal and an antigen molecule. The method is a universal antigen self-assembled aluminum adjuvant delivery platform, which is non-selective for the charge of the antigen and can conveniently construct a self-assembled particle vaccine by binding the Fc affinity tag commonly used in antigen purification as an assembly element.

### SUMMARY

The present invention provides an aluminum nanocrystal vaccine delivery system and a preparation method thereof, and a self-assembled particle adjuvant vaccine based on specific binding of an Fc affinity protein covering a surface of an aluminum nanocrystal in the aluminum nanocrystal vaccine delivery system and a vaccine antigen molecule. The aluminum nanocrystal according to the present invention can be effectively bound to the Fc affinity protein through a modified molecule so as to construct the aluminum nanocrystal delivery system, and can effectively carry immune antigens to further construct the self-assembled particle adjuvant vaccine, so that the stability of carrying the antigens can be remarkably improved, the density of the antigens carried on the surface of the aluminum nanocrystal is increased, and the immune antigens can be effectively delivered to lymph node tissues; and moreover, the cellular internalization of the immune antigens is greatly enhanced, immune cells are activated extremely efficiently, and thus the antibody generation level is enhanced.

In order to achieve above objective of the present invention, the present invention provides the following technical solution:
the present invention provides an aluminum nanocrystal delivery system constructed by effectively binding an aluminum nanocrystal to an Fc affinity protein through a modified molecule.
the average particle size of the aluminum nanocrystal is 5-500 nm; and the surface of the aluminum nanocrystal carries the modified molecule that can be linked to a group of the Fc affinity protein through host-guest coordination and chemoselectivity covalent modification.

Further, the aluminum nanocrystal is prepared by the following steps:
adding a modified molecule solution to an aluminum salt solution, and stirring and fully mixing to obtain a mixed solution; and
adding the mixed solution to an alkaline solution to obtain a reaction solution, adjusting the pH of the reaction solution to be 5.5-8.0, then reacting, standing, centrifuging and taking precipitate to obtain the aluminum nanocrystal.

The aluminum salt is more than one of aluminum chloride, aluminum nitrate, aluminum sulfate, and aluminum acetate; the solvent used in the aluminum salt solution is pure water, or a sodium acetate solution with the concentration of 0.01 mol/L; the concentration of the aluminum salt is 0.01-0.5 mol/L; and the stirring speed is 100-1,400 rpm, and the stirring time is 10 min to 5 h.

The modified molecule is more than one of L-O-phosphoserine, citric acid, oxalic acid, salicylic acid, and ATP; the concentration of the modified molecule solution is 0.01-0.5 mol/L; and in the mixed solution, the molar ratio of the aluminum salt to the modified molecules is (10:1)-(1:10).

The alkaline solution is more than one of NaOH, KOH and ammonia solution; the concentration of the alkaline solution is 0.01-0.5 mol/L; and the rate of adding the alkaline solution to the mixed solution is 1-20 mL/min.

Further, the reaction temperature is 25-90°C; the reaction time is 10 min to 10 h; and the standing time is 1-12 h.

Further, the modified molecule includes one that can perform amino, carboxyl and sulfydryl functional group functionalization and chemoselectivity covalent modification group functionalization on a surface functional group of the aluminum nanocrystal.

Furthermore, the modified molecule is specifically more than one of 1-(3-dimethylaminopropyl)-3-ethylcarbodiimide hydrochloride, maleimide, succinimide, azide and alkyne.

Further, the corresponding modified molecule includes a corresponding reactant that can perform covalent binding with the Fc affinity protein, such as N-hydroxysuccinimide (NHS) obtained from 1-(3-dimethylaminopropyl)-3-ethylcarbodiimide hydrochloride (EDC)/(N-hydroxysuccinimide) NHS reaction.

The present invention also provides a preparation method for an aluminum nanocrystal delivery system. The preparation method includes the following steps:
(1) adding an aluminum nanocrystal and a modified molecule to a solvent, and stirring the mixture to allow reaction, thereby obtaining a mixed solution 1;
(2) adding an Fc affinity protein and a modified molecule to a solvent, and stirring the mixture to allow reaction, thereby obtaining a mixed solution 2; and
(3) uniformly mixing the mixed solution 1 in step (1) and the mixed solution 2 in step (2), then reacting, centrifugally washing, taking the precipitate, and resuspending to obtain the aluminum nanocrystal delivery system.

Specifically, the solvent is specifically injection water, ultrapure water, normal saline, a Tris buffer solution, or a phosphate buffer solution; the mass-to-volume ratio of the aluminum nanocrystal to the solvent is 0.5-60 mg/mL; the molar ratio of aluminum ions to the modified molecule in the aluminum nanocrystal is 1:(0.001-1000);
in step (1), the stirring reaction temperature is 4-25°C, the stirring reaction time is 0.5-24 h, and the stirring rate of the stirring reaction is 100-600 rpm;
in step (2), the stirring reaction temperature is 4-25°C, and the stirring reaction time is 0.5-24 h; and
in step (3), the reaction time is 0.5-24 h, the centrifugal washing is performed twice, and the product is resuspended into the buffer solution with the pH of 7.4-9.

Based on that, the present invention also provides a self-assembled particle adjuvant vaccine based on binding of an aluminum nanocrystal delivery system and an antigen molecule, wherein the aluminum nanocrystal delivery system is the abovementioned aluminum nanocrystal delivery system, and/or the aluminum nanocrystal delivery system is obtained by the abovementioned preparation method, and specifically, the surface of the aluminum nanocrystal prepared by the abovementioned method is covered with an Fc affinity protein and a specifically bound self-assembled vaccine antigen molecule.

The Fc affinity protein is a G protein and/or an A protein with high affinity to an Fc region; the G protein and/or A protein is a bacterial protein or a recombinant protein;
the bacterial protein is derived from Group G Streptococcus and Staphylococcus aureus;
the recombinant protein is arbitrarily modified by gene recombination, but still maintains the Fc affinity, and specifically is a recombinant A protein/G protein, an A protein or G protein with C-terminal recombinant cysteine or phosphoserine, and a polypeptide fragment derived from the A protein and/or G protein; and
the polypeptide fragment from the A protein and/or G protein is recombinant peptide formed by different amino acid sequences and chemically synthesized polypeptide sequences.

Specifically, the Fc affinity protein is derived from the species of human, murine, rabbits, pigs and sheep.

The binding mode of the Fc affinity protein and the aluminum nanocrystal may be that the aluminum nanocrystal is bound with the Fc affinity protein in a non-covalent mode, such as electrostatic or host-guest coordination adsorption, or covalent modification, chemoselectivity specific binding and other forms.

The vaccine antigen molecule includes one or more of an Fc fragment containing pathogenic subunit antigen, recombinant subunit antigen, antigen epitope peptide and nucleic acid antigen.

The pathogen includes viruses, bacteria and/or parasites; and
Preferably, the viruses are selected from DNA virus and/or RNA virus.

More preferably, the viruses are selected from coronaviridae, herpesviridae, rhabdoviridae, filoviridae, orthomyxoviridae, paramyxoviridae, picornaviridae, hepadnaviridae, flaviviridae, papillomaviridae, poxviridae and retroviridae.

Further preferably, the viruses are selected from novel coronavirus, influenza virus, herpes simplex virus, vesicular stomatitis virus, vaccinia virus, HIV and HBV.

Preferably, the bacteria are selected from gram-positive bacteria and/or gram-negative bacteria.

More preferably, the bacteria are selected from Streptococcus pneumoniae, Haemophilus influenzae, salmonella, Neisseria meningitidis, Staphylococcus epidermidis, Staphylococcus aureus, Escherichia coli, Klebsiella Pneumoniae, Klebsiella oxytoca, Enterobacter cloacae, Citrobacter frazier, Pesudomonas pyocyaneum, Acinetobacter baumannii, Mycobacterium tuberculosis and Helicobacter pylori.

Further preferably, the bacteria are selected from Streptococcus pneumoniae, salmonella, Neisseria meningitidis, Staphylococcus aureus, Escherichia coli, Klebsiella oxytoca, Enterobacter cloacae, Helicobacter pylori and the like.

The parasites are selected from one or more of plasmodium, toxoplasma gondii, trypanosome, schistosome, filariasis and leishmania.

The present invention also provides a preparation method of a self-assembled particle adjuvant vaccine based on binding of the aluminum nanocrystal delivery system and the vaccine antigen molecule, specifically, the method includes: uniformly mixing an Fc fragment containing vaccine antigen molecule with an aluminum nanocrystal delivery system solution, so as to obtain a self-assembled particle vaccine based on an aluminum nanocrystal with a surface covered with an Fc affinity protein and an antigen.

Specifically, materials are mixed by a rotary shaker at 4-25°C for 10-60 min so as to be uniformly mixed; and the concentration of the aluminum nanocrystal is 0.5-60 mg/mL, the concentration of antigen is 5-200 mg/mL, and the mass ratio of the vaccine antigen molecules to aluminum ions in the aluminum nanocrystal is 1:(0.1-100).

An administration method of the self-assembled particle adjuvant vaccine includes oral perfusion, intravenous injection, intramuscular injection, intraperitoneal injection and any combination thereof.

More preferably, the administration method of the adjuvant vaccine bound reagent is intramuscular injection.

The present invention has the technical effects that:
The aluminum nanocrystal provided by the present invention can be effectively bound with the Fc affinity protein and effectively carry the immune antigen, thus obtaining more excellent immunotherapy effect with lower antigen dosage and lower injection dosage; and the immune cells are efficiently activated to implement balanced body fluid and cell immunity.

### BRIEF DESCRIPTION OF THE DRAWINGS

In order to more clearly illustrate the embodiments of the present application or the technical solutions in the prior art, the drawings required for use in the embodiments or the description of the prior art are briefly introduced below.
FIG. 1 shows TEM images of aluminum nanocrystals obtained according to Examples 1, 2 and 3 of the present invention;
FIG. 2 shows an XRD image of an aluminum nanocrystal obtained according to an Example 1 of the present invention;
FIG. 3 shows BMDCs activation level and MHC expression level by an adjuvant according to an Example 17 of the present invention;
FIG. 4 shows RBD specific IgG antibody titer in mouse serum 19, 35 and 56 days after vaccination according to an Example 19 of the present invention;
FIG. 5 shows pseudovirus neutralizing antibody titer in mouse serum 56 days after vaccination according to an Example 19 of the present invention; and
FIG. 6 is a schematic diagram of aluminum nanocrystal surface Fc binding protein modification and antigen self-assembly process according to the present invention.

### DETAILED DESCRIPTION

The present invention discloses an aluminum nanocrystal vaccine delivery system and a preparation method thereof, and a self-assembled particle adjuvant vaccine based on specific binding of an Fc affinity protein covering a surface of an aluminum nanocrystal in the aluminum nanocrystal vaccine delivery system and a vaccine antigen molecule. Those skilled in the art may refer to the content herein with appropriate modification of the process parameters. It is particularly to be noted that all similar substitutions and modifications are obvious to those skilled in the art and are considered to be included in the present invention. The methods and applications of the present invention have been described through preferred embodiments, and the skill in the art can obviously modify or appropriately change and combine the methods and applications described herein without departing from the disclosure, spirit and scope of the present invention to implement and apply the technology of the present invention.

The aluminum nanocrystal vaccine delivery system and the preparation method thereof, and the self-assembled particle adjuvant vaccine based on specific binding of the Fc affinity protein covering the surface of the aluminum nanocrystal in the aluminum nanocrystal vaccine delivery system and the vaccine antigen molecule, and raw materials or reagents used in the application of vaccine immune synergy can be commercially purchased.

The aluminum nanocrystal adjuvant used in the examples of the present invention is homemade, and the experimental solution is shown in the Examples.

The present invention is further described below in conjunction with the Examples:

### Example 1

AlCl₃·6H₂O was dissolved in 0.01 mol/L of NaAc to prepare an aluminum salt solution containing 0.055 mol/L of aluminum ion; the obtained aluminum salt solution and 0.05 mol/L of L-O-phosphoserine (purchased from Sigma-Aldrich, CAS No. 407-41-0) were stirred for 2 h at a stirring speed of 300 rpm; after fully mixing, 0.1 mol/L of NaOH solution was added at a speed of 7 ml/min through a peristaltic pump; and the pH of the mixed reaction solution was adjusted to be about 7. The molar ratio of Al³⁺ to the L-O-phosphoserine was 1:2; and the reaction temperature was 35°C, and the stirring reaction was carried out for 2 h; then, standing was allowed for 7 h after the reaction was finished; centrifuging followed by washing was performed; and finally sterilizing by high-pressure steam or by a filter membrane was performed to prepare an aluminum nanocrystal.

### Example 2

AlCl₃·6H₂O was dissolved in 0.01 mol/L of NaAc to prepare an aluminum salt solution containing 0.45 mol/L of aluminum ion; the obtained aluminum salt solution and 0.35 mol/L of sodium citrate were stirred for 5 h at a stirring speed of 100 rpm; after fully mixing, 0.01 mol/L of NaOH solution was added at a speed of 1 ml/min through a peristaltic pump; and the pH of the mixed reaction solution was adjusted to be about 5.5. The molar ratio of Al³⁺ to the sodium citrate was 1:6; and the reaction temperature was 45°C, and the stirring reaction was carried out for 5 h; then, standing was allowed for 1 h after the reaction was finished; centrifuging followed by washing was performed; and finally sterilizing by high-pressure steam or by a filter membrane was performed to prepare an aluminum nanocrystal.

### Example 3

AlCl₃·6H₂O was dissolved in pure water to prepare an aluminum salt solution containing 0.01 mol/L of aluminum ion; the obtained aluminum salt solution and 0.02 mol/L of salicylic acid were stirred for 5 h at a stirring speed of 200 rpm; after fully mixing, 0.5 mol/L of ammonia solution was added at a speed of 9 ml/min through a peristaltic pump; and the pH of the mixed reaction solution was adjusted to be about 7.5. The molar ratio of Al³⁺ to the salicylic acid was 7:1; and the reaction temperature was 25°C, and the stirring reaction was carried out for 10 h; then, standing was allowed for 5 h after the reaction was finished; centrifuging followed by washing was performed; and finally sterilizing by high-pressure steam or by a filter membrane was performed to prepare an aluminum nanocrystal.

### Comparative Example 1

AlCl₃·6H₂O was dissolved in 0.01 mol/L of NaAc to prepare an aluminum salt solution containing 0.5 mol/L of aluminum ion; then, 0.15 mol/L of NaOH solution was added at a speed of 7 ml/min through a peristaltic pump; and the pH of the mixed reaction solution was adjusted to be about 7. The reaction temperature was 90°C, the stirring reaction was carried out for 10 min, and the stirring speed was 900 rpm; then, standing was allowed at room temperature for 5 h after the reaction was finished; centrifuging followed by washing was performed; and finally sterilizing by high-pressure steam or by a filter membrane was performed to prepare aluminum nanocrystal without modification molecules.

### Example 4

Aluminum sulfate was dissolved in 0.01 mol/L of NaAc to prepare an aluminum salt solution containing 0.45 mol/L of aluminum ion; the obtained aluminum salt solution and 0.35 mol/L of sodium citrate were stirred for 3 h at a stirring speed of 800 rpm; after fully mixing, 0.01 mol/L of KOH solution was added at a speed of 1 ml/min through a peristaltic pump; and the pH of the mixed reaction solution was adjusted to be about 5.5. The molar ratio of Al³⁺ to the sodium citrate was 1:6; and the reaction temperature was 45°C, and the stirring reaction was carried out for 5 h; then, standing was allowed for 1 h after the reaction was finished; centrifuging followed by washing was performed; and finally sterilizing by high-pressure steam or by a filter membrane was performed to prepare an aluminum nanocrystal.

### Example 5

Aluminum nitrate was dissolved in pure water to prepare an aluminum salt solution containing 0.01 mol/L of aluminum ion; the obtained aluminum salt solution and 0.02 mol/L of aqueous ATP were stirred for 5 h at a stirring speed of 1,000 rpm; after fully mixing, 0.5 mol/L of ammonia solution was added at a speed of 9 ml/min through a peristaltic pump; and the pH of the mixed reaction solution was adjusted to be about 7.5. The molar ratio of Al³⁺ to the salicylic acid was 7:1; and the reaction temperature was 25°C, and the stirring reaction was carried out for 10 h; then, standing was allowed for 5 h after the reaction was finished; centrifuging followed by washing was performed; and finally sterilizing by high-pressure steam or by a filter membrane was performed to prepare an aluminum nanocrystal.

### Example 6

AlCl₃·6H₂O was dissolved in 0.01 mol/L of NaAc to prepare an aluminum salt solution containing 0.5 mol/L of aluminum ion; the obtained aluminum salt solution and 0.01 mol/L of oxalic acid were stirred for 10 min at a stirring speed of 1,400 rpm; after fully mixing, 0.15 mol/L of ammonia solution was added at a speed of 10 ml/min through a peristaltic pump; and the pH of the mixed reaction solution was adjusted to be about 5. The reaction temperature was 90°C, and the stirring reaction was carried out for 10 h; then, standing was allowed at room temperature for 1 h after the reaction was finished; centrifuging followed by washing was performed; and finally sterilizing by high-pressure steam or by a filter membrane was performed to prepare an aluminum nanocrystal.

### Example 7

Aluminum acetate was dissolved in 0.01 mol/L of NaAc to prepare an aluminum salt solution containing 0.25 mol/L of aluminum ion; the obtained aluminum salt solution and 0.5 mol/L of ATP were stirred for 40 min at a stirring speed of 1,200 rpm; after fully mixing, 0.05 mol/L of KOH solution was added at a speed of 20 ml/min through a peristaltic pump; and the pH of the mixed reaction solution was adjusted to be about 8. The reaction temperature was 55°C, and the stirring reaction was carried out for 2 h; then, standing was allowed at room temperature for 12 h after the reaction was finished; centrifuging followed by washing was performed; and finally sterilizing by high-pressure steam or by a filter membrane was performed to prepare an aluminum nanocrystal.

### Example 8

### (1) Physicochemical property characterization of aluminum nanocrystal

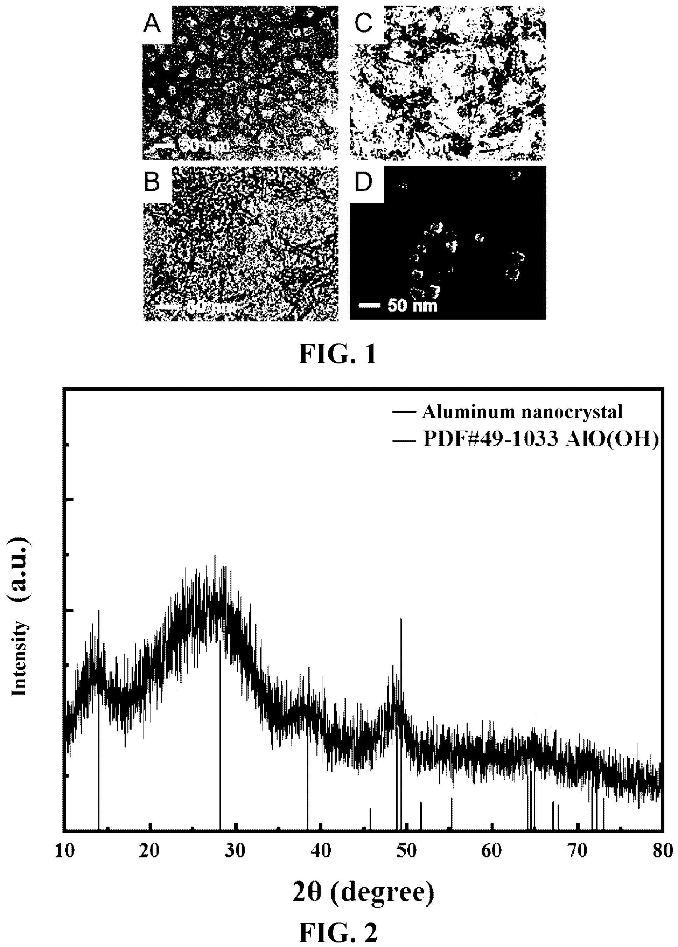

An aluminum nanocrystal was diluted to be 10 µg/ml at 25°C, and then dripped on a common carbon support membrane, so as to observe the structure of the aluminum nanocrystal through an electron microscope, and the aluminum nanocrystal was displayed as particles under a transmission electron microscope (FEI company, model: Tecnai G2 20S-TWIN). As shown in FIG. 1, FIG. 1 shows TEM images of the nanoparticles obtained when the aluminum element and the modified molecule were in different molar ratios in Example 1. FIG. 1A shows an electron microscope image of the product in the Example 1, FIG. 1B shows an electron microscope image of the product without a modified molecule in the Comparative Example 1, FIG. 1C shows an electron microscope image of the product in the Example 2, and FIG. 1D shows an electron microscope image of the product in the Example 3.

As shown in FIG. 1, when the modified molecule exists, the obtained aluminum nanocrystal adjuvant has smaller average size. The average size of the aluminum nanocrystals obtained in the Example 1 is 20 nanometers, the average size of the aluminum nanocrystals obtained in the Example 2 is 57.1 nanometers, and the average size of the aluminum nanocrystals obtained in the Example 3 is 25.3 nanometers. When the modified molecule does not exist, the obtained products are clustered aggregated particles with the average size of about 350 nanorods. Therefore, in the presence of the modified molecule, the obtained aluminum nanocrystal has more uniform size, better dispersity and better solution stability.

### (2) XRD characterization of aluminum nanocrystal

10 mL of the aluminum nanocrystal prepared in the Example 1 was subjected to freeze-drying to obtain a powder sample, XRD characterization was performed on the powder sample, and then the powder sample was analyzed with Jade, as shown in FIG. 2. FIG. 2 shows an XRD image of product nanoparticles obtained in the Example 1.

As shown in FIG. 2, the obtained aluminum nanocrystal can be correspondingly of an aluminum hydroxide structure.

### (3) Particle size and surface charge characterization for aluminum nanocrystal

The aluminum nanocrystals prepared in the Examples 1-7 and the aluminum nanocrystal without the modified molecule prepared in the Comparative Example 1 were respectively diluted to be 10 µg/ml at 25°C, and the particle size and Zeta potential off the aluminum nanocrystals were tested by a nano particle analyzer(purchased from Malvern company, model: Zetasizer Nano ZS). The result is shown in Table 1.

The physicochemical properties of the aluminum nanocrystal particles prepared in the Examples 1-7 and the aluminum nanocrystal without modified molecules prepared in the Comparative Example 1 are shown in Table 1.

**Table 1 Physicochemical properties of two aluminum nanocrystals obtained with and without modified molecule**

| Test items | Example 1 Aluminum nanocrystal particles | Example 2 Aluminum nanocrystal particles | Example 3 Aluminum nanocrystal particles | Comparative Example 1 Aluminum nanocrystal particles | Example 4 Aluminum nanocrystal particles | Example 5 Aluminum nanocrystal particles | Example 6 Aluminum nanocrystal particles | Example 7 Aluminum nanocrystal particles |
|---|---|---|---|---|---|---|---|---|
| Average hydrated particle size | 33.7nm | 71.4nm | 31.6nm | 451.6nm | 76.2nm | 5.6nm | 158.2nm | 482.1nm |
| Potential (mV) | -23 | -16 | -14 | 26 | -18 | -21 | -17 | -13 |

As shown in Table 1, the aluminum nanocrystals that were nano-sized and uniformly dispersed were prepared in the Examples 1 to 7 and Comparative Example 1.

### Example 9

A preparation method for an aluminum nanocrystal delivery system with a surface covered with an Fc affinity protein:
(1) The aluminum nanocrystal obtained in the Example 1 and 1-(3-dimethylaminopropyl)-3-ethylcarbodiimide hydrochloride (EDC) were added into normal saline, wherein the concentration of the aluminum nanocrystal was 10 mg/ml, and the concentration of EDC was 0.02 mol/L; reaction was carried out at the temperature of 25°C for 8 h while stirring at a rotating speed of 500 rpm, so as to obtain a solution I; and the molar ratio of EDC to aluminum element was 1:100 finally; and
(2) the Fc affinity protein (SPA, Article No. P6031, Staphylococcus aureus protein A, purchased from Sigma-Aldrich (Shanghai)) and N-hydroxysuccinimide (NHS) were mixed according to a molar ratio of 1:5, added to an aqueous solution, and stirred to react for 12 h to obtain a solution II.

The solution II was added to the solution I, so that the ratio of the added NHS to EDC was 1:1.2, then reaction was continued at the temperature of 25°C for 8 h; and after centrifugally washing twice, the reaction product was re-suspended into a phosphate buffer solution with the pH of 7.4, thereby obtaining the aluminum nanocrystal delivery system solution with the surface covered with the Fc affinity protein (SPA).

### Example 10

A preparation method for an aluminum nanocrystal delivery system with a surface covered with an Fc affinity protein:
(1) The aluminum nanocrystal obtained in the Example 1 and 1-(3-dimethylaminopropyl)-3-ethylcarbodiimide hydrochloride (EDC) were added into normal saline, wherein the concentration of the aluminum nanocrystal was 10 mg/ml, and the concentration of EDC was 0.1 mol/L; reaction was carried out at the temperature of 25°C for 10 h while stirring at a rotating speed of 1,000 rpm, so as to obtain a solution I; and the molar ratio of EDC to aluminum element was 1: 1 finally; and
(2) the Fc affinity protein and N-hydroxysuccinimide (NHS) were mixed according to a molar ratio of 1:5, added to an aqueous solution, and stirred to react at temperature of 4°C for 12 h to obtain a solution II.

The solution II was added to the solution I, so that the ratio of the added NHS to EDC was 1:1.2, then reaction was continued at the temperature of 4°C for 8 h; and after centrifugally washing twice, the reaction product was re-suspended into a phosphate buffer solution with the pH of 7.4, thereby obtaining the aluminum nanocrystal delivery system solution with the surface covered with the Fc affinity protein.

### Example 11

A preparation method for an aluminum nanocrystal delivery system with a surface covered with an Fc affinity protein:
(1) The aluminum nanocrystal obtained in the Example 1 and 1-(3-dimethylaminopropyl)-3-ethylcarbodiimide hydrochloride (EDC) were added into normal saline, wherein the concentration of the aluminum nanocrystal was 10 mg/ml, and the concentration of EDC was 0.06 mol/L; reaction was carried out at the temperature of 4°C for 6 h while stirring at a rotating speed of 400 rpm, so as to obtain a solution I; and the molar ratio of EDC to aluminum element was 10:1 finally; and
(2) the Fc affinity protein and N-hydroxysuccinimide (NHS) were mixed according to a molar ratio of 1:20, added to an aqueous solution, and stirred to react at temperature of 25°C for 12 h to obtain a solution II.

The solution II was added to the solution I, so that the ratio of the added NHS to EDC was 11:1, then reaction was continued at the temperature of 10°C for 8 h; and after centrifugally washing twice, the reaction product was re-suspended into a phosphate buffer solution with the pH of 7.4, thereby obtaining the aluminum nanocrystal delivery system solution with the surface covered with the Fc affinity protein.

### Example 12

A preparation method for an aluminum nanocrystal delivery system with a surface covered with an Fc affinity protein:
(1) The aluminum nanocrystal obtained in the Example 4 and 1-(3-dimethylaminopropyl)-3-ethylcarbodiimide hydrochloride (EDC) were added into normal saline, wherein the concentration of the aluminum nanocrystal was 10 mg/ml, and the concentration of EDC was 0.1 mol/L; reaction was carried out at the temperature of 25°C for 0.5 h while stirring at a rotating speed of 1,000 rpm, so as to obtain a solution I; and the molar ratio of EDC to aluminum element was 1: 1000 finally; and
(2) the streptococcus Fc affinity protein G (Article No.: S29820, purchased from Shanghai Yuanye Biotechnology Co ., Ltd) and maleimide were mixed according to a molar ratio of 1:5, added to an aqueous solution, and stirred to react at temperature of 4°C for 24 h to obtain a solution II.

The solution II was added to the solution I, so that the ratio of the added maleimide to EDC was 1:1, then reaction was continued at the temperature of 20°C for 0.5 h; and after centrifugally washing twice, the reaction product was re-suspended into a phosphate buffer solution with the pH of 7.4, thereby obtaining the aluminum nanocrystal delivery system solution with the surface covered with the Fc affinity protein.

### Example 13

A preparation method for an aluminum nanocrystal delivery system with a surface covered with an Fc affinity protein:
(1) The aluminum nanocrystal obtained in the Example 5 and 1-(3-dimethylaminopropyl)-3-ethylcarbodiimide hydrochloride (EDC) were added into pure water, wherein the concentration of the aluminum nanocrystal was 10 mg/ml, and the concentration of EDC was 0.06 mol/L; reaction was carried out at the temperature of 4°C for 24 h while stirring at a rotating speed of 400 rpm, so as to obtain a solution I; and the molar ratio of EDC to aluminum element was 100: 10 finally; and
(2) the C-terminal recombinant cysteine Fc affinity protein A and succinimide were mixed according to a molar ratio of 1:20, added to an aqueous solution, and stirred to react at temperature of 25°C for 2 h to obtain a solution II.

The solution II was added to the solution I, so that the ratio of the added succinimide to EDC was 2:1; then reaction was continued at the temperature of 15°C for 3 h; and after centrifugally washing twice, the reaction product was re-suspended into a phosphate buffer solution with the pH of 7.4, thereby obtaining the aluminum nanocrystal delivery system solution with the surface covered with the Fc affinity protein.

### Example 14

A preparation method for an aluminum nanocrystal delivery system with a surface covered with an Fc affinity protein:
(1) The aluminum nanocrystal obtained in the Example 6 and 1-(3-dimethylaminopropyl)-3-ethylcarbodiimide hydrochloride (EDC) were added into a Tris buffer solution, wherein the concentration of the aluminum nanocrystal was 10 mg/ml, and the concentration of EDC was 0.06 mol/L; reaction was carried out at the temperature of 15°C for 6 h while stirring at a rotating speed of 400 rpm, so as to obtain a solution I; and the molar ratio of EDC to aluminum element was 20:1 finally; and
(2) the C-terminal recombinant phosphoserine Fc affinity protein G and N-hydroxysuccinimide (NHS) were mixed according to a molar ratio of 1:20, added to the Tris buffer solution, and stirred to react at temperature of 25°C for 0.5 h to obtain a solution II.

The solution II was added to the solution I, so that the ratio of the added NHS to EDC was 1:2, then reaction was continued at the temperature of 4°C for 24 h; and after centrifugally washing twice, the reaction product was re-suspended into a phosphate buffer solution with the pH of 9, thereby obtaining the aluminum nanocrystal delivery system solution with the surface covered with the Fc affinity protein.

### Example 15

A preparation method for an aluminum nanocrystal delivery system with a surface covered with an Fc affinity protein:
The aluminum nanocrystal obtained in the Example 7 was added to injection water to obtain a solution I, wherein the concentration of the aluminum nanocrystal was 10 mg/ml; and an Fc affinity protein polypeptide fragment was added to the injection water solution to obtain a solution II. The solution II was added to the solution I, thus the ratio of the added affinity protein to aluminum element was 1: 100; reaction was continued at 25°C for 24 h, so that the aluminum nanocrystals were bound together by host-guest coordination and electrostatic adsorption; and after centrifugally washing twice, the reaction product was re-suspended into a phosphate buffer solution with the pH of 7.4, so as to obtain the aluminum nanocrystal delivery system solution with the surface covered with the Fc affinity protein.

### Example 16

Characterization of protein assembly capability of aluminum nanocrystal delivery system:
In order to verify the assembly capability of adjuvants prepared by the Example 9-15 for Fc-containing protein, the assembly capability was quantitatively tested in this example with a fluorescent tag IgG . The aluminum nanocrystal delivery system solution was diluted to be 1 mg/ml (0.1 ml by volume) by a phosphate buffer; the pH was adjusted to be about 7; then the diluted solution was uniformly mixed with 10 µg of FITC-IgG(Goat Anti-Mouse IgG H&L(FITC), purchased from abcam with the Article No.: ab6785) phosphate buffer in equal volume, and then the mixture was stood at room temperature for 1 h; and centrifuging was carried out to collect the supernatant; the 520nm fluorescence intensity was measured; and then the assembly efficiency was calculated according to the following formula: adsorption rate (%)=(10 µg FITC-IgG fluorescence intensity-supernatant fluorescence intensity)/FITC-IgG fluorescence intensity × 100%.

The assembly efficiency of the aluminum nanocrystal delivery system is shown in Table 2.

**Table 2**

| Test items | Example 9 | Example 10 | Example 11 | Example 12 | Example 13 | Example 14 | Example 15 |
|---|---|---|---|---|---|---|---|
| Protein assembly capability % | >99% | >90% | >83% | >90% | >83% | >86% | >89% |

The nanocrystal delivery system obtained in the Example 9 has the best assembly performance for the Fc-containing proteins, so the nanocrystal delivery system obtained in the Example 9 was selected in the subsequent examples.

### Example 17

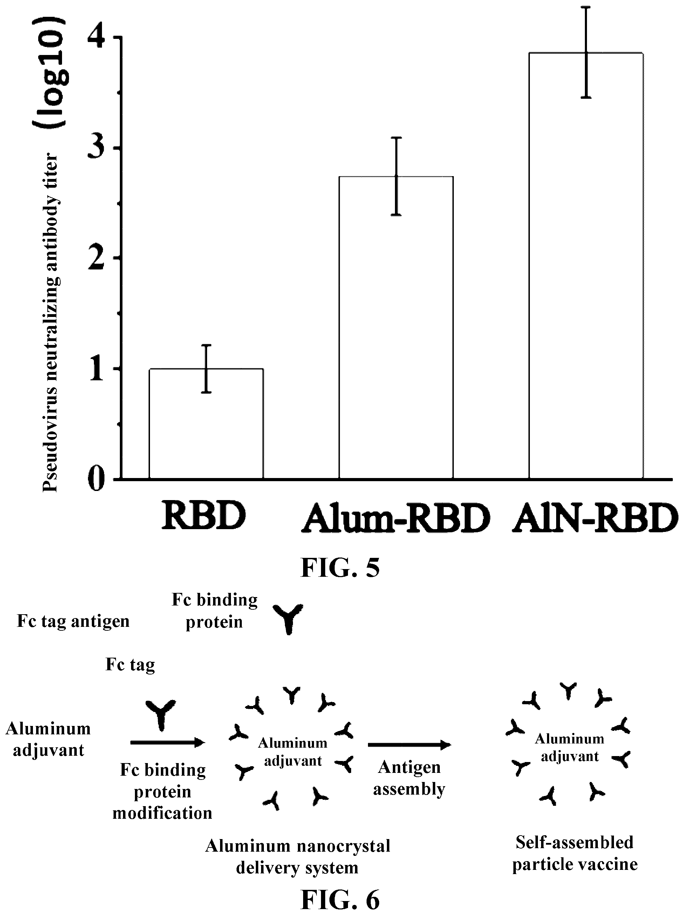

An assembling method of a self-assembled particle adjuvant vaccine of an aluminum nanocrystal delivery system and an Fc fragment-containing antigen molecule is shown in FIG. 6.

10 µg of Fc fragment-containing novel coronavirus recombinant RBD antigen (purchased from Sino Biological, Article No.: 40591-V05H1) was added to 100 µl of 1 mg/ml aluminum nanocrystal delivery system solution obtained in the Example 9 while stirring at room temperature; affinity self-assembly was performed in a buffer solution with the pH of 8.5; suction was repeated for 100 times by an injector for uniform mixing; after that, the mixture was mixed in a rotary shaker for 30 min, thereby obtaining the self-assembled particle adjuvant vaccine based on the aluminum nanocrystal delivery system and the Fc fragment-containing neocoronavirus recombinant RBD subunit antigen molecule.

### Example 18

Evaluation of BMDCs activation and antigen presentation capability of self-assembled particle adjuvant vaccine

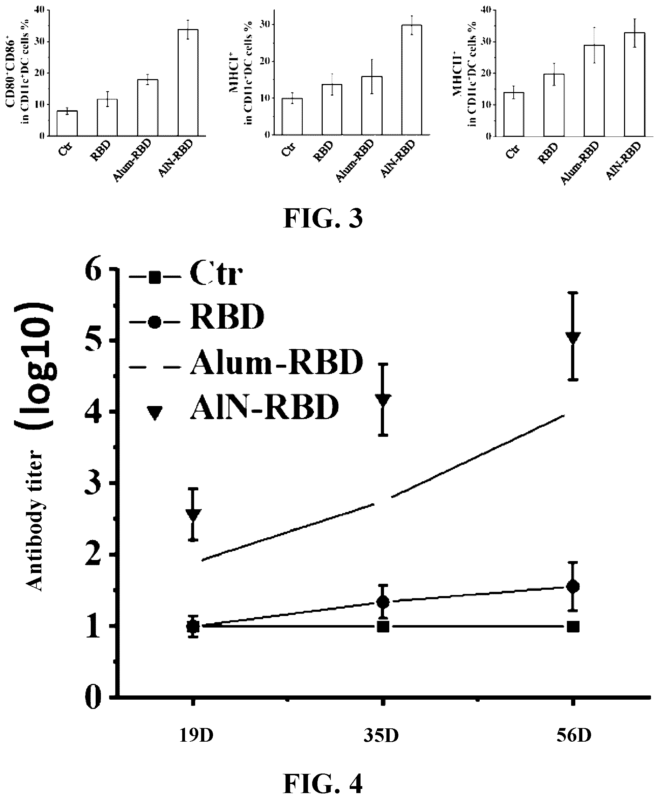

BMDC cells were inoculated in a six-well plate at a density of 3 × 10⁵ cells in each well, and grown overnight. Then the cells were grouped, and 100 µl of treatment liquid was respectively added: (1), normal saline (Ctr group); (2), 10 µg of novel coronavirus recombinant RBD antigen (RBD group); (3), commercial aluminum adjuvant Alum and 10 µg of novel coronavirus recombinant RBD antigen (Alum-RBD group); and (4), cells treated with self-assembled particle adjuvant vaccine (AlN-RBD group) prepared in the Example 17. Further incubation was carried out for 24 h, the cells were collected, and then stained with anti-CD11c, anti-CD80 and anti-CD86, anti-MHC-1 and anti-MHC-II flow cytometry staining solutions. The expression levels of co-stimulatory factors CD80 and CD86 and antigen recognition signals MHC-1 and MHC-II on the surfaces of BMDCs were determined by a flow cytometer, as shown in FIG. 3. FIG. 3 shows evaluation of BMDCs activation and antigen presentation capability of the self-assembled particle adjuvant vaccine obtained in the Example 17.

As shown in FIG. 3, compared with the commercial aluminum adjuvant, the self-assembled particle adjuvant vaccine has a better BMDC activation effect and higher antigen presentation capability.

### Example 19

Inoculation of self-assembled particle adjuvant vaccine constructed in the Example 17.
A) Under the premise of following the national animal health protocol, BALB/c mice aged 6-8 weeks were selected for vaccination 3 times, with 5 mice in each group, and a total of five groups, namely (1) Ctr group: control group (normal saline); (2) Ag group: 10 µg RBD antigen; (3) Alum-Ag group: 50 µg of aluminum adjuvant carrying 10 µg of RBD antigen (Invivogen, Alhydrogel^{®} adjuvant 2%, CAS: 21645-51-2); and (4) AlN-Ag group: self-assembled particle adjuvant vaccine constructed in the Example 17, that is, aluminum nanocrystal-carrying system particle group carrying 10 µg of RBD antigen (Ag). The first injection on mouse thigh muscle was performed on day 0, the second vaccination was performed on day 21, and serum samples were collected on days 19, 35 and 56.
B) The IgG level in the mouse serum induced by the vaccine in step A was evaluated by a conventional enzyme-linked immunosorbent assay (ELISA). First, the mouse serum was serially diluted in equal proportions; the diluted serum was added to a 96-well ELISA plate pre-coated with RBD antigen (2 µg/ml) and stood at 37°C for 2 h; after washing, diluted HRP-conjugated goat anti-mouse IgG antibody (dilution 1:2000) was added, 100 µl per well. Incubation was performed at 37°C for 1.5 h; after washing, TMB colorimetric solution was added, incubated together, and a stop solution was added to stop the reaction; and the absorbance at OD450 was read using an ELISA reader. The specific IgG antibody titer is shown in FIG. 4. FIG. 4 shows an evaluation of the antibody response caused by the self-assembled particle adjuvant vaccine constructed in the Example 17.

As shown in FIG. 4, the self-assembled particle adjuvant vaccine can effectively enhance the antibody response caused by the novel coronavirus vaccine in mice. At the same antigen dose, the antibody titer level of the self-assembled particle adjuvant vaccine far exceeds that of the commercial aluminum adjuvant group, the pure antigen group and the control group.

### Example 20

Serum samples from mice 56 days after vaccination with the vaccine obtained in step A of the Example 19 were subjected to a pseudovirus infection neutralization test, and the specific process was as follows: pre-incubating supernatant containing pseudovirus (50 µl; purchased from Sino Biologic company, Article No.: PSV001) and continuously diluted mice serum at 37°C for 1 h, and then adding the mixture to 293T cells (5×104 cells) expressing ACE2. A fresh culture medium was added after 24 h, and then the cells were lysed by a commercially available cell lysis buffer solution. After a luciferase substrate was added, the relative luciferase activity was measured in a luminometer (Bio-Tech). The pseudovirus neutralization efficiency was calculated and expressed as 50% of neutralization antibody titer. The settings of the RBD group, the Alum-RBD group and the AlN-RBD group are shown in the Example 10.

FIG. 5 shows serum neutralization effect of a nano vaccine drug constructed in the Example 17 against the pseudovirus; and as shown in FIG. 5, the nano vaccine drug obtained in the Example 17 can still induce significantly enhanced neutralization antibody response against novel coronavirus even under the condition of relatively low antibody carrying amount.

### Example 21

10 µg of Fc fragment-containing virus recombinant antigen (such as herpes virus, varicella stomatitis virus, vaccinia virus, HIV and HBV) was added to 100 µl of 1 mg/ml aluminum nanocrystal delivery system solution obtained in the Example 12 while stirring at room temperature; affinity self-assembly was carried out in a buffer solution with the pH of 8.2; suction was repeated for 100 times by an injector for uniform mixing; the mixture was mixed in a rotary shaker for 30 min, thereby obtaining the self-assembled particle adjuvant vaccine based on the aluminum nanocrystal delivery system and the Fc fragment-containing virus recombinant subunit antigen molecule. The antiviral activity of the constructed self-assembled particle adjuvant vaccine was evaluated according to the method in the Example 18, with result shown in a Table 3.

**Table 3 Antiviral activity of self-assembled particle adjuvant vaccine**

| Test items | Herpes virus | Varicella stomatitis virus | Vaccinia virus | HIV | HBV |
|---|---|---|---|---|---|
| CD80⁺CD86⁺ Cell proportion | 38% | 42% | 32% | 34% | 41% |

According to the data in Table 3, the constructed self-assembled particle adjuvant vaccine has a good immune activation effect.

### Example 22

10 µg of Fc fragment-containing OVA was added to 100 µl of 1 mg/ml solution of the aluminum nanocrystal delivery system obtained in the Example 13 while stirring at room temperature; affinity self-assembly was performed in a buffer solution with the pH of 8.0; suction was repeated for 100 times by an injector for uniform mixing; the mixture was mixed in a rotary shaker for 30 min to obtain the self-assembled particle adjuvant vaccine based on the aluminum nanocrystal delivery system and the Fc fragment-containing OVA. Mice infected with Streptococcus pneumoniae, salmonella, Neisseria meningitidis, Staphylococcus aureus, Escherichia coli, Enterobacter cloacae, Helicobacter pylori and the like were subjected to immunization treatment by administrating in an oral gavage immunization mode with PBS (phosphate buffer solution) being used as a blank control group; the anti-OVA IgG and anti-OVA S-IgA levels generated in the mice (the antiviral activity of the constructed self-assembled particle adjuvant vaccine) were detected according to the method in the Example 20; and the result is shown in Table 4.

**Table 4 Detection result of anti-OVA IgG level and anti-OVA S-IgA level**

| Test items | Streptococcus pneumoniae | Salmonella | Neisseria meningitidis | Staphylococcus aureus | Enterobacter cloacae | Escherichia coli | Helicobacter pylori |
|---|---|---|---|---|---|---|---|
| Anti-OVA IgG/Blank control (ug/ml) | 28.2/0.7 | 27.4/0.5 | 25.110.4 | 29.6/0.6 | 30.4/0.9 | 27.1 /0.5 | 29.2/0.7 |
| Anti-OVA S-IgA/Blank control (mg/ml) | 4.1/0.2 | 6.2/0.3 | 7.0/0.5 | 4.8/0.4 | 4.5/0.3 | 6.5/0.7 | 5.4/0.3 |

According to the data in Table 4, the constructed self-assembled particle adjuvant vaccine has a better immune activation effect.

### Example 23

10 µg of Fc fragment-containing parasite recombinant antigen (such as plasmodium, toxoplasma gondii, trypanosome, schistosome, filariasis and leishmania) was added to 100 µl of 1 mg/ml aluminum nanocrystal delivery system solution obtained in the Example 14 while stirring at room temperature; affinity self-assembly was performed in a buffer solution with the pH of 8.0; suction was repeated for 100 times for uniform mixing, the mixture was mixed in a rotary shaker for 30 min, thereby obtaining the self-assembled particle adjuvant vaccine based on the aluminum nanocrystal delivery system and the Fc fragment-containing parasite recombinant subunit antigen molecule. Schizont infected mice were administrated with the vaccine in an intraperitoneal injection manner, enhanced immune administration was performed twice on Day 20 and Day 40, mouse serum was taken out on Day 14 after the last administration, and the concentration of the corresponding antibody was detected via ELISA. The result is shown in Table 5.

**Table 5 Detection result of concentration of antibody in mouse serum**

| Test items | Plasmodium | Toxoplasma gondii | Trypanosome | Schistosome | Leishmania | Filariasis |
|---|---|---|---|---|---|---|
| Vaccine/Blank antibody OD | 3.5/1.0 | 3.2/0.8 | 4.0/1.3 | 3.8/0.9 | 3.4/1.2 | 4.6/1.5 |

According to the data in Table 5, the constructed self-assembled particle adjuvant vaccine has a better immune activation effect.

The above are only preferred embodiments of the present application. It is to be noted that for those skilled in the art, several improvements and modifications can be made without departing from the principles of the present application. These improvements and modifications should also be regarded as the scope of protection of the present application.

## Claims

1. An aluminum nanocrystal delivery system, wherein the aluminum nanocrystal delivery system is constructed by effectively binding an aluminum nanocrystal to an Fc affinity protein through a modified molecule.

2. The aluminum nanocrystal delivery system according to claim 1, wherein the average particle size of the aluminum nanocrystal is 5-500 nm; and the modified molecule that can be linked to a group of the Fc affinity protein through host-guest coordination and chemoselectivity covalent modification is carried on the surface of the aluminum nanocrystal.

3. The aluminum nanocrystal delivery system according to claim 2, wherein the aluminum nanocrystal is prepared by the following steps:
fully mixing an aluminum salt solution and a modified molecule solution to obtain a mixed solution; and
adding an alkaline solution to the mixed solution to obtain a reaction solution, adjusting the reaction solution to reach preset pH, then reacting, standing, centrifuging and taking precipitate to obtain the aluminum nanocrystal.

4. The aluminum nanocrystal delivery system according to claim 3, wherein a solute of the aluminum salt solution is more than one of aluminum chloride, aluminum nitrate, aluminum sulfate, and aluminum acetate; the solvent used in the aluminum salt solution is pure water, or a sodium acetate solution with the concentration of 0.01 mol/L; the concentration of the aluminum salt solution is 0.01-0.5 mol/L; and the stirring speed is 100-1400 rpm, and the stirring time is 10 min to 5 h;
the modified molecule is more than one of L-O-phosphoserine, citric acid, oxalic acid, salicylic acid, and ATP; the concentration of the modified molecule solution is 0.01-0.5 mol/L; and in the mixed solution, the molar ratio of the aluminum salt to the modified molecule is (10:1)-(1:10);
the alkaline solution is more than one of NaOH, KOH and ammonia solution; the concentration of the alkaline solution is 0.01-0.5 mol/L; the rate of adding the alkaline solution to the mixed solution is 1-20 mL/min; the pH is 5.0-8.0; the reaction temperature is 25-90°C; the reaction time is 10 min to 10 h; and the standing time is 1-12 h.

5. The aluminum nanocrystal delivery system according to claim 1, wherein the modified molecule comprises a modified molecule that can perform amino, carboxyl and sulfydryl functional group functionalization and chemoselectivity covalent modification group functionalization on a surface functional group of the aluminum nanocrystal.

6. The aluminum nanocrystal delivery system according to claim 5, wherein the modified molecule is selected from more than one of 1-(3-dimethylaminopropyl)-3-ethylcarbodiimide hydrochloride, maleimide, succinimide, azide and alkyne.

7. A preparation method of an aluminum nanocrystal delivery system according to claim 1, comprising the following steps:
(1) adding an aluminum nanocrystal and a modified molecule to a solvent, and stirring the mixture to allow reaction, thereby obtaining a mixed solution 1;
(2) adding an Fc affinity protein and a modified molecule to a solvent, and stirring the mixture to allow reaction, thereby obtaining a mixed solution 2; and
(3) uniformly mixing the mixed solution 1 in step (1) and the mixed solution 2 in step (2), reacting, centrifugally washing, taking the precipitate, and resuspending to obtain the aluminum nanocrystal delivery system.

8. The preparation method of an aluminum nanocrystal delivery system according to claim 7, wherein the solvent is selected from injection water, ultrapure water, a Tris buffer solution, or a phosphate buffer solution and normal saline; the mass-to-volume ratio of the aluminum nanocrystal to the solvent is 0.5-60 mg/mL; the molar ratio of aluminum ions to the modified molecules in the aluminum nanocrystal is 1:(0.001-1000);
in step (1), the stirring reaction temperature is 4-25°C, the stirring reaction time is 0.5-24 h, and the stirring rate of the stirring reaction is 100-600 rpm;
in step (2), the stirring reaction temperature is 4-25°C, and the stirring reaction time is 0.5-24 h; and
in step (3), the reaction time is 0.5-24 h, the centrifugal washing is performed twice, and the product is resuspended into the buffer solution with the pH of 7.4-9.

9. An self-assembled particle adjuvant vaccine based on binding of an aluminum nanocrystal delivery system and a vaccine antigen molecule, wherein the aluminum nanocrystal delivery system is the aluminum nanocrystal delivery system according to any one of claims 1 to 6, and/or the aluminum nanocrystal delivery system is obtained by the preparation method according to any one of clams 7 to 8; and the surface of the aluminum nanocrystal is covered with the Fc affinity protein and the specifically bound self-assembled vaccine antigen molecule.

10. The self-assembled particle adjuvant vaccine based on binding of the aluminum nanocrystal delivery system and the vaccine antigen molecule according to claim 11, wherein the Fc affinity protein is a G protein and/or A protein with high affinity to an Fc region; and the vaccine antigen molecule comprises one or more of an Fc fragment containing pathogenic subunit antigen, a recombinant subunit antigen, an antigen epitope peptide and a nucleic acid antigen.

11. The self-assembled particle adjuvant vaccine based on binding of the aluminum nanocrystal delivery system and the vaccine antigen molecule according to claim 10, wherein the G protein and/or A protein is a bacterial protein or a recombinant protein.

12. The self-assembled particle adjuvant vaccine based on binding of the aluminum nanocrystal delivery system and the vaccine antigen molecule according to claim 11, wherein the bacterial protein is a bacterial protein from Group G Streptococcus and/or Staphylococcus aureus.

13. The self-assembled particle adjuvant vaccine based on binding of the aluminum nanocrystal delivery system and the vaccine antigen molecule according to claim 11, wherein the recombinant protein is a recombinant A protein or G protein, an A protein or G protein with C-terminal recombinant cysteine or phosphoserine, and a polypeptide fragment derived from the A protein and/or G protein.

14. The self-assembled particle adjuvant vaccine based on binding of the aluminum nanocrystal delivery system and the vaccine antigen molecule according to claim 10, wherein the pathogen comprises viruses, bacteria and/or parasites.

15. The self-assembled particle adjuvant vaccine based on binding of the aluminum nanocrystal delivery system and the vaccine antigen molecule according to claim 14, wherein the viruses are selected from DNA virus and/or RNA virus.

16. The self-assembled particle adjuvant vaccine based on binding of the aluminum nanocrystal delivery system and the vaccine antigen molecule according to claim 15, wherein the viruses are selected from coronaviridae, herpesviridae, rhabdoviridae, filoviridae, orthomyxoviridae, paramyxoviridae, picornaviridae, hepadnaviridae, flaviviridae, papillomaviridae, poxviridae and retroviridae.

17. The self-assembled particle adjuvant vaccine based on binding of the aluminum nanocrystal delivery system and the vaccine antigen molecule according to claim 16, wherein the viruses are selected from novel coronavirus, influenza virus, herpes simplex virus, vesicular stomatitis virus, vaccinia virus, HIV and HBV.

18. The self-assembled particle adjuvant vaccine based on binding of the aluminum nanocrystal delivery system and the vaccine antigen molecule according to claim 14, wherein the bacteria are selected from gram-positive bacteria and/or gram-negative bacteria.

19. The self-assembled particle adjuvant vaccine based on binding of the aluminum nanocrystal delivery system and the vaccine antigen molecule according to claim 18, wherein the bacteria are selected from Streptococcus pneumoniae, Haemophilus influenzae, salmonella, Neisseria meningitidis, Staphylococcus epidermidis, Staphylococcus aureus, Escherichia coli, Klebsiella Pneumoniae, Klebsiella oxytoca, Enterobacter cloacae, Citrobacter frazier, Pesudomonas pyocyaneum, Acinetobacter baumannii, Mycobacterium tuberculosis and Helicobacter pylori.

20. The self-assembled particle adjuvant vaccine based on binding of the aluminum nanocrystal delivery system and the vaccine antigen molecule according to claim 19, wherein the bacteria are selected from Streptococcus pneumoniae, salmonella, Neisseria meningitidis, Staphylococcus aureus, Escherichia coli, Klebsiella oxytoca, Enterobacter cloacae, Helicobacter pylori and the like.

21. The self-assembled particle adjuvant vaccine based on binding of the aluminum nanocrystal delivery system and the vaccine antigen molecule according to claim 14, wherein the parasites are selected from one or more of plasmodium, toxoplasma gondii, trypanosome, schistosome, filariasis and leishmania.

22. A preparation method of the self-assembled particle adjuvant vaccine based on binding of the aluminum nanocrystal delivery system and the vaccine antigen molecule according to claim 9 or 10, comprising uniformly mixing a Fc fragment-containing vaccine antigen molecule with an aluminum nanocrystal delivery system solution, so as to obtain a self-assembled particle vaccine based on an aluminum nanocrystal with a surface covered with an Fc affinity protein and an antigen.
